(19) Europäisches Patentamt
European Patent Office
Office européen des brevets

(11) **EP 4 180 063 A1**

(12) **EUROPEAN PATENT APPLICATION**
published in accordance with Art. 153(4) EPC


(43) Date of publication:
**17.05.2023 Bulletin 2023/20**

(21) Application number: **21838191.1**

(22) Date of filing: **06.07.2021**

(51) International Patent Classification (IPC):
***A61L 2/10*** *(2006.01)* ***A61L 2/24*** *(2006.01)*
***H01L 33/00*** *(2010.01)*

(52) Cooperative Patent Classification (CPC):
**A61L 2/10; A61L 2/24; H01L 33/00**

(86) International application number:
**PCT/JP2021/025506**

(87) International publication number:
**WO 2022/009897 (13.01.2022 Gazette 2022/02)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **09.07.2020 JP 2020118799**



(71) Applicant: **Asahi Kasei Kabushiki Kaisha**
**Tokyo 1000006 (JP)**

(72) Inventors:
- **KODAMA, Atsushi**
  **Tokyo 100-0006 (JP)**
- **YABUKI, Naoto**
  **Tokyo 100-0006 (JP)**
- **OTSUKI, Shunya**
  **Tokyo 100-0006 (JP)**

(74) Representative: **Hoffmann Eitle**
**Patent- und Rechtsanwälte PartmbB**
**Arabellastraße 30**
**81925 München (DE)**


(54) **ULTRAVIOLET IRRADIATION APPARATUS AND ULTRAVIOLET IRRADIATION METHOD**


(57) There is provided an ultraviolet irradiation apparatus that prevents exposure to ultraviolet light more reliably. The ultraviolet irradiation apparatus includes: a UVC-LED (12a) configured to emit ultraviolet light; a biosensor (11) disposed in the vicinity of the UVC-LED (12a) such that an irradiation area of the UVC-LED (12a) and a detection area of the biosensor (11) overlap each other, the biosensor (11) configured to detect the presence of a person; a control circuit (1a) configured to control the UVC-LED (12a) based on an output signal of the biosensor (11); and a light shielding cover (3b) configured to shield a part of the ultraviolet light emitted from the UVC-LED (12a). The control circuit (1a) is configured to drive and control the UVC-LED (12a) to stop emission of the ultraviolet light when the biosensor (11) has detected the presence of the person, and to emit the ultraviolet light again when a state has switched from a state where the biosensor (11) is detecting the presence of the person to a state where the biosensor (11) does not detect the presence of the person.

FIG. 5A

HUMAN SENSOR
UVC-LED
T1
T2
T3
T2
T1


EP 4 180 063 A1

FIG. 5B

HUMAN SENSOR
UVC-LED
T2
T3

## Description

### Technical Field

The present invention relates to an ultraviolet irradiation apparatus and an ultraviolet irradiation method.

### Background Art

Since ultraviolet light has sterilization capability, various ultraviolet irradiation apparatus as sterilization apparatus have been proposed.

In recent years, LEDs (light emitting diodes) capable of emitting a wavelength that can perform sterilization have been put to practical use, and this makes it possible to realize an apparatus configuration that cannot be realized with an apparatus using a bulb as an ultraviolet light source, so that small portable ultraviolet irradiators have been proposed.

Further, if a human body enters an ultraviolet irradiation area provided by an ultraviolet irradiation apparatus, there is a risk of exposure to harmful ultraviolet light, and therefore there are also proposed ultraviolet irradiation apparatus or the like that prevent the exposure by combining a human sensor and a control circuit that controls the irradiation timing of ultraviolet light (e.g., see PTL 1).

### Citation List

### Patent Literature

PTL 1: JP 2017-29293 A

### Summary of Invention

### Technical Problem

However, with the ultraviolet irradiation apparatus that performs the irradiation in the open system like the conventional ultraviolet irradiation apparatus that prevents the exposure as described above, an area to be irradiated with ultraviolet light occurs even outside the detection range of the human sensor, and therefore it is difficult to reliably prevent the exposure to the ultraviolet light.

Accordingly, an ultraviolet irradiation apparatus that can prevent the exposure more reliably has been desired.

Therefore, the present invention has been made by focusing on the conventional unsolved problem described above and has an object to provide an ultraviolet irradiation apparatus and an ultraviolet irradiation method that can prevent exposure to ultraviolet light more reliably.

### Solution to Problem

An ultraviolet irradiation apparatus according to one embodiment of the present invention includes: a light source configured to emit ultraviolet light; a biosensor disposed in the vicinity of the light source such that an irradiation range of the light source and a detection range of the biosensor overlap each other, the biosensor configured to detect a presence of a person; an electronic circuit configured to control the light source based on an output signal of the biosensor; and a light shielding cover configured to shield a part of the ultraviolet light emitted from the light source, wherein: the light source is disposed in an area on a side opposite to one side where an irradiation target of the ultraviolet light is present, the light source and the irradiation target sandwiching the light shielding cover; a sensing surface of the biosensor and a light emitting surface of the light source are disposed to face in the same direction; an angle $\theta 2$ half a maximum detection angle of the biosensor in a state where the biosensor is incorporated in the ultraviolet irradiation apparatus is greater than a maximum angle $\Theta 1$ formed by a normal vector of the light emitting surface and the ultraviolet light emitted from the light source and passing without being shielded by the light shielding cover; and the electronic circuit is configured to control the light source to stop emission of the ultraviolet light when the biosensor has detected the presence of the person, the electronic circuit configured to control the light source to emit the ultraviolet light again when a state has switched from a state where the biosensor is detecting the presence of the person to a state where the biosensor does not detect the presence of the person.

An ultraviolet irradiation method according to another embodiment of the present invention includes: a light source configured to emit ultraviolet light; and a biosensor disposed in the vicinity of the light source such that an irradiation range of the light source and a detection range of the biosensor overlap each other, the biosensor configured to detect a presence of a person, the ultraviolet irradiation method including: disposing the light source in an area on a side opposite to one side where an irradiation target of the ultraviolet light is present, the light source and the irradiation target sandwiching a light shielding cover configured to shield a part of the ultraviolet light emitted from the light source, and disposing a sensing surface of the biosensor and a light emitting surface of the light source to face in the same direction; and shielding the ultraviolet light such that an angle half a maximum detection angle of the biosensor in a disposed state is greater than a maximum angle formed by a normal vector of the light emitting surface of the light source and the ultraviolet light emitted from the light source and passing without being shielded by the light shielding cover.

The maximum detection angle of the biosensor in the disposed state, referred to herein, refers to a maximum detection angle detectable by the biosensor in the state where the biosensor, the light source, and the light shielding cover are disposed to be able to perform ultraviolet irradiation. For example, when the biosensor is disposed on the same side as the light source with respect to the light shielding cover, and the detection range of the biosensor is narrowed by the light shielding cover, a maximum value of a detection angle in the narrowed state is a maximum detection angle, while, when the biosensor is disposed on the same side as the light source, but the detection range is not narrowed by the light shielding cover, a maximum value of a detection angle in the non-narrowed state, i.e., a viewing angle determined by the specification of the biosensor, is a maximum detection angle.

Advantageous Effects of Invention

According to one aspect of the present invention, it is possible to more reliably prevent exposure to ultraviolet light while performing ultraviolet irradiation.

Brief Description of Drawings

FIG. 1A is a perspective view illustrating an example of an ultraviolet irradiation apparatus according to a first embodiment of the present invention;
FIG. 1B is a see-through diagram of FIG. 1A;
FIG. 2 is a sectional view illustrating an A-A' section of FIG. 1A;
FIG. 3A is a front view of the ultraviolet irradiation apparatus, FIG. 3B is a rear view thereof, and FIG. 3C is a side view thereof;
FIG. 4 is a block diagram illustrating an example of the ultraviolet irradiation apparatus according to the second embodiment of the present invention;
FIGS. 5A and 5B are examples of timing charts for describing the operation of the ultraviolet irradiation apparatus;
FIG. 6 is a sectional view illustrating an A-A' section in a modification of FIG. 1A;
FIGS. 7A and 7B are perspective views illustrating another example of the ultraviolet irradiation apparatus;
FIG. 8 is a diagram illustrating an example of installation of the ultraviolet irradiation apparatus;
FIGS. 9A and 9B are diagrams for describing the operation of an ultraviolet irradiation apparatus according to a second embodiment;
FIGS. 10A and 10B are diagrams for describing the operation of an ultraviolet irradiation apparatus according to a third embodiment;
FIG. 11 is another example of the ultraviolet irradiation apparatus according to the third embodiment;
FIG. 12 is an explanatory diagram for describing the operation of an ultraviolet irradiation apparatus according to a fourth embodiment;
FIGS. 13A and 13B are explanatory diagrams for describing the operation of an ultraviolet irradiation apparatus according to a fifth embodiment;
FIG. 14 is an explanatory diagram for describing the operation of an ultraviolet irradiation apparatus according to a sixth embodiment;
FIG. 15 is an explanatory diagram for describing the operation of an ultraviolet irradiation apparatus according to a seventh embodiment; and
FIG. 16 is an example of the simulation results.

Description of Embodiments

Next, embodiments of the present invention will be described with reference to the drawings. In the following description of the drawings, the same or like signs are given to the same or like portions. However, the drawings are only exemplary, and the relationship between the thickness and the plane dimensions, the ratio of the thicknesses of layers, and the like differ from actual ones. Further, portions having different dimensional relationships and ratios are included also between the drawings.

The embodiments given below merely exemplify apparatus and methods for embodying the technical idea of the present invention, and the technical idea of the present invention does not limit the arrangements and the like of constituent components to those described below. The technical idea of the present invention can be changed in various ways within the technical scope defined by the claims.

<First Embodiment>

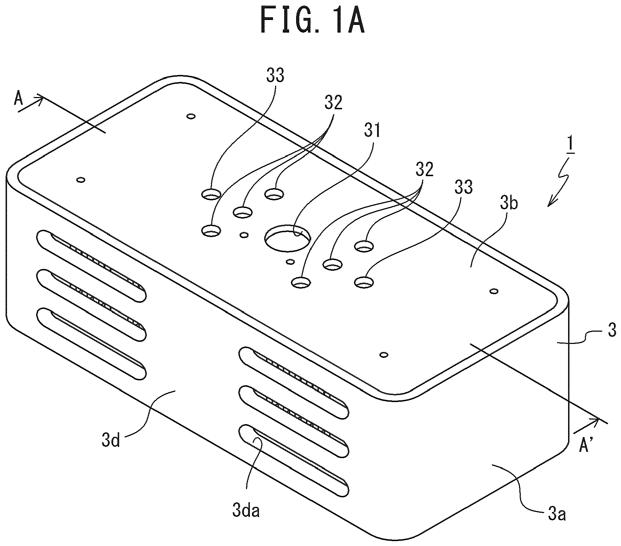

FIGS. 1A to 4 are diagrams illustrating an example of an ultraviolet irradiation apparatus according to a first embodiment of the present invention.

An ultraviolet irradiation apparatus 1 performs sterilization of a place that many people contact, such as, for example, an ATM of a bank.

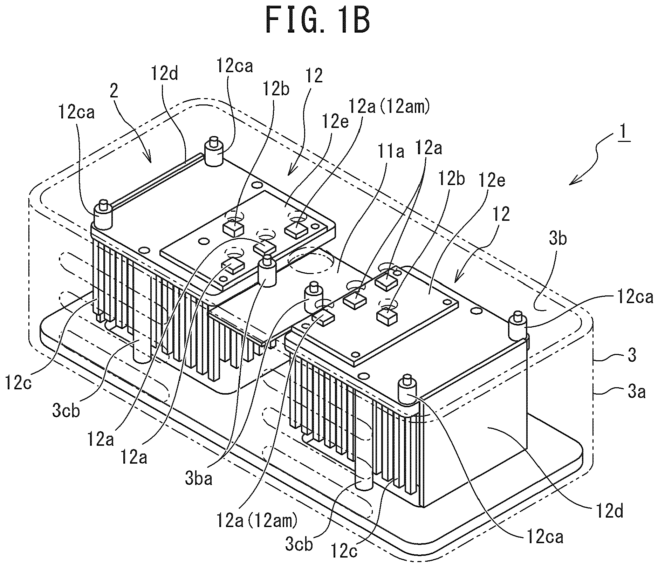

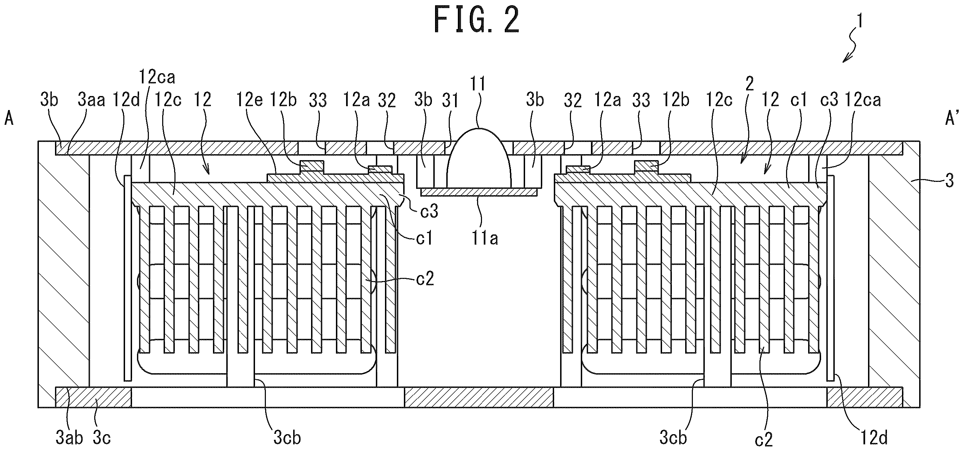

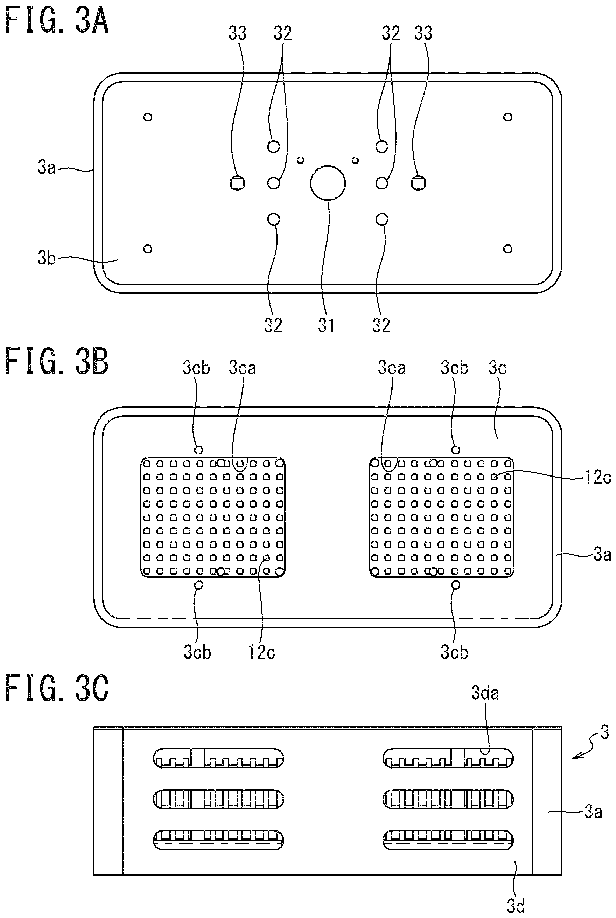

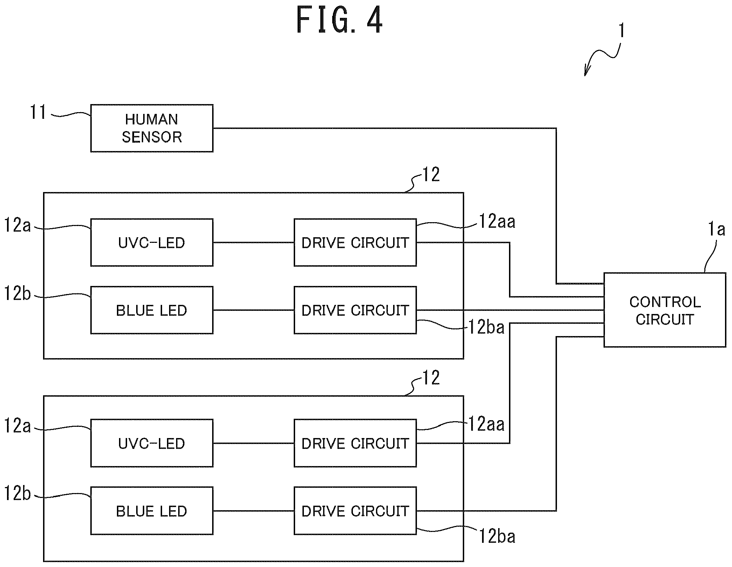

FIG. 1A is a perspective view illustrating an example of the external appearance of the ultraviolet irradiation apparatus 1, and FIG. 1B is a perspective view illustrating an example of seeing through the inside of an outer case 3. FIG. 2 is an A-A' sectional view of FIG. 1A, FIG. 3A is a front view of the ultraviolet irradiation apparatus 1, FIG. 3B is a rear view thereof, and FIG. 3C is a top view thereof, wherein a bottom view is identical to the top view. FIG. 4 is a block diagram illustrating a functional configuration of the ultraviolet irradiation apparatus 1. In FIG. 1A, a later-described biosensor 11 is omitted.

The ultraviolet irradiation apparatus 1 includes an apparatus body 2 and the outer case 3 having a generally rectangular parallelepiped shape and housing the apparatus body 2.

The apparatus body 2 includes the single biosensor 11 and two sets of light emitting units 12. The light emitting unit 12 includes three UVC-LEDs (light sources) 12a, a single blue LED (visible light source) 12b, a heat sink 12c, and a driver board 12d. The UVC-LED (light source) 12a is a light emitting diode that emits ultraviolet light with a peak wavelength of 200 nm or more and 300 nm or less, and in terms of the efficiency of sterilization of bacteria or the like, the peak wavelength is more preferably 255 nm or more and 280 nm or less and further preferably 260 nm or more and 270 nm or less. The driver board 12d is mounted with a drive circuit 12aa that drives and controls the UVC-LEDs 12a, and with a drive circuit 12ba that drives and controls the blue LED 12b. The driver board 12d of either one of the two sets of the light emitting units 12 is mounted with the drive circuit 12aa for the UVC-LEDs 12a and the drive circuit 12ba for the blue LED 12b and further mounted with a control circuit (electronic circuit) 1a. The control circuit 1a receives a detection signal (output signal) of the biosensor 11, controls, based on the detection signal, the UVC-LEDs 12a and the blue LEDs 12b via the respective drive circuits 12aa, 12ba, and controls the entire ultraviolet irradiation apparatus 1.

The biosensor 11 is formed by, for example, a quantum-type infrared sensor (IR sensor) and mounted on a sensor board 11a for the biosensor 11. The UVC-LEDs 12a and the blue LED 12b are mounted on each of light emitting boards 12e. The light emitting boards 12e are provided separately from the sensor board 11a and thus are not in contact with the sensor board 11a so as not to be thermally connected to the sensor board 11a.

The light emitting board 12e has a generally rectangular shape. The three UVC-LEDs 12a are disposed at regular intervals at positions close to one of long sides of the light emitting board 12e along the one of the long sides, and the blue LED 12b is disposed at a position at an approximately central portion of the light emitting board 12e rather close to the other long side.

A sensing surface of the biosensor 11 and light emitting surfaces of all the UVC-LEDs 12a are disposed to face in the same direction. Further, all the UVC-LEDs 12a are disposed in a distance of 20 mm or less from the biosensor 11 in plan view. By making the distance between the UVC-LED 12a and the biosensor 11 short, it is possible to limit an ultraviolet irradiation area to within a biosensor detection area even in the vicinity of the UVC-LED 12a being the light source.

The biosensor 11 is not limited to the quantum-type infrared sensor (IR sensor), and a pyroelectric-type infrared sensor, a microwave radar, a reflective-type infrared sensor, a millimeter-wave radar, an ultrasonic sensor, a temperature detection sensor, a ToF sensor, or the like may also be applied.

The biosensor 11, the UVC-LEDs 12a, the blue LEDs 12b, and the driver boards 12d are connected via wiring (not illustrated).

The outer case 3 includes a case body 3a having a rectangular parallelepiped shape and being open at its front and rear surfaces corresponding to front and rear surfaces of the ultraviolet irradiation apparatus 1, a light shielding cover 3b provided at the opening of the case body 3a on the front surface side, and a rear surface cover 3c provided at the opening of the case body 3a on the rear surface side. As illustrated in FIG. 2, a stepped portion 3aa for supporting the light shielding cover 3b and a stepped portion 3ab for supporting the rear surface cover 3c are formed at opening ends of the case body 3a, respectively.

As illustrated in FIG. 3C, slit holes 3da are formed in each of two side surfaces 3d of the case body 3a contacting long sides of the case body 3a, the side surfaces 3d serving as top and bottom surfaces of the ultraviolet irradiation apparatus 1. The slit holes 3da are formed along the longitudinal direction of the outer case 3 such that the slit holes 3da are formed in three rows in the height direction of the case body 3a and in two rows in the longitudinal direction of the case body 3a.

As illustrated in FIG. 3A, the light shielding cover 3b is formed with a single circular hole 31 for the biosensor 11, six circular holes 32 for the UVC-LEDs 12a, and two circular holes 33 for the blue LEDs 12b.

The hole 31 is formed at the center of the light shielding cover 3b and has a diameter greater than those of the other holes 32, 33. The hole 32 is about 1/3 to 1/4 of the hole 31 in diameter, and the hole 33 is slightly larger than the hole 32.

The holes 32 and the holes 33 are arranged half and half to sandwich the hole 31 from both sides along the longitudinal direction of the light shielding cover 3b so as to be symmetric with respect to the hole 31. In this example, the three holes 32 are arranged at regular intervals along the width direction (the lateral direction of the light shielding cover 3b), and the hole 32 located at the center and the hole 33 are located on the center line in the longitudinal direction (the longitudinal direction of the light shielding cover 3b).

As illustrated in FIG. 1B, two boss-like projections 3ba for fixing the sensor board 11a are formed on a back surface of the light shielding cover 3b being a surface on the side facing the rear surface cover 3c. The projections 3ba are provided at positions that are spaced apart from each other on both sides of the hole 31 along the longitudinal direction and are slightly offset to one side from the longitudinal direction center line. By threading screws into the projections 3ba from the back surface side of the sensor board 11a in the state where the sensor board 11a is abutted against the projections 3ba, the sensor board 11a is disposed at a position spaced apart from the light shielding cover 3b by the length of the projections 3ba.

Further, boss-like projections 12ca for restraining top surfaces of the heat sinks 12c are provided at positions on the back surface of the light shielding cover 3b slightly spaced apart from four corners of the back surface of the light shielding cover 3b toward its center side.

As illustrated in FIG. 3B, the rear surface cover 3c is formed with two generally rectangular openings 3ca for heat dissipation of the heat sinks 12c, and the rectangular openings 3ca are formed at an interval along the longitudinal direction at an approximately central portion of the rear surface cover 3c in the width direction. Further, in the vicinity of each of the openings 3ca on a surface of the rear surface cover 3c on the side facing the light shielding cover 3b, boss-like fixing members 3cb for fixing the heat sink 12c are provided at positions respectively at two long sides of the opening 3ca that are slightly closer to a short side of the rear surface cover 3c compared to central portions of the long sides of the opening 3ca.

The heat sink 12c has a frog shape formed with a large number of rod-like members c2 on one of surfaces of a flat plate portion c1 of, for example, a generally square shape. The driver board 12d is fixed to a flange portion c3 of the heat sink 12c along one of four side surfaces formed by each of the rod-like members c2. On the side opposite to the driver board 12d on a surface of the flat plate portion c1 on the side opposite to the rod-like members c2, the light emitting board 12e is fixed to the flange portion c3 such that the UVC-LEDs 12a mounted on the light emitting board 12e are arranged along a side of the flat plate portion c1.

Then, the heat sinks 12c to which the driver boards 12d and the light emitting boards 12e are fixed are disposed so that the light emitting board 12e sides are closer to the central portion, and screws are threaded from the sides of the heat sinks 12c opposite to the rod-like members c2 in the state where the rod-like member c2 sides face the rear surface cover 3c, so that the flange portions c3 and the fixing members 3cb are fixed together. Consequently, the heat sinks 12c and the rear surface cover 3c are fixed together. Further, by threading screws into the projections 12ca from the side of the light shielding cover 3b opposite to the sensor board 11a in the state where the light shielding cover 3b to which the sensor board 11a is fixed is disposed with the sensor board 11a side facing inward, the light shielding cover 3b and the case body 3a are integrated so that the apparatus body 2 is fixed in the outer case 3. With this structure, it is possible to provide a state where the heat sinks 12c and the biosensor 11 are not thermally connected. That is, when the thermal resistance from the heat sink 12c to the biosensor 11 is 2.5 K/W or more, the heat transfer from the UVC-LEDs 12a to the biosensor 11 can be ignored so that it can be regarded as not being thermally connected.

The heat sink 12c is not limited to the frog-shaped heat sink, and a heat sink including a plurality of fins may also be applied.

In the state where the apparatus body 2 is fixed in the outer case 3, the biosensor 11 is disposed to slightly protrude from the surface of the light shielding cover 3b so as to be able to detect a person who is present near an irradiation target of the ultraviolet irradiation apparatus 1. The UVC-LEDs 12a and the blue LEDs 12b are positioned so that their respective irradiation lights are emitted to the outside of the outer case 3 through the corresponding holes 32, 33 and further that irradiation areas by the UVC-LEDs 12a and irradiation areas by the blue LEDs 12b almost coincide with each other.

The ultraviolet irradiation apparatus 1 operates using, for example, a 24 V DC voltage input to an input terminal (not illustrated) as a power supply voltage. As illustrated in FIG. 4, the control circuit 1a that controls the entire ultraviolet irradiation apparatus 1 drives and controls the UVC-LEDs 12a via the drive circuits 12aa of the two light emitting units 12 and the blue LEDs 12b via the drive circuits 12ba thereof based on a detection signal of the biosensor 11 so that the ultraviolet irradiation apparatus 1 performs ultraviolet irradiation on an irradiation target while avoiding ultraviolet irradiation on a human body.

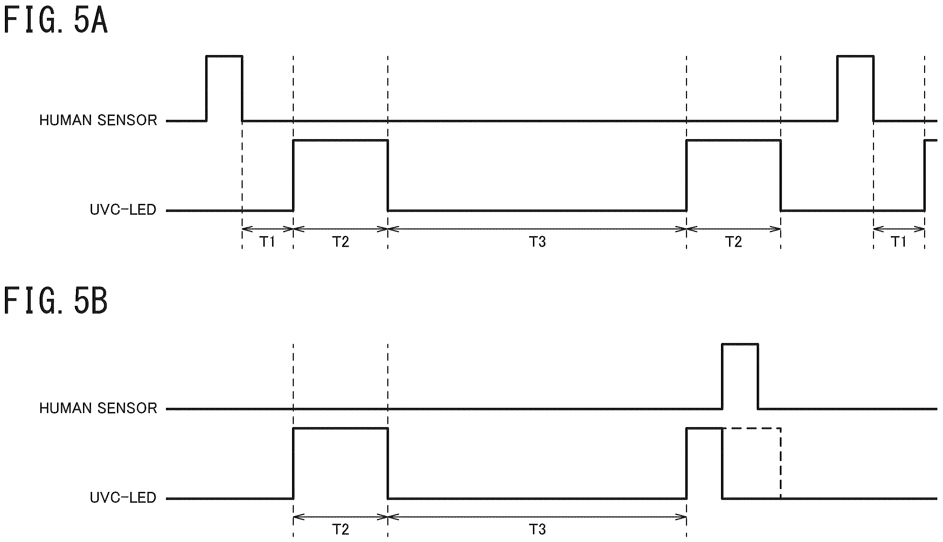

Specifically, the control circuit 1a detects, based on a detection signal of the biosensor 11, switching from a state detecting a person to a state detecting no person. Then, as illustrated in a timing chart of FIG. 5A, when the switching from the state detecting the person to the state detecting no person is detected, at a time point when a preset waiting time T1 has elapsed, the UVC-LED 12a is driven to perform ultraviolet irradiation for an irradiation time T2. Then, when the irradiation time T2 has elapsed, the ultraviolet irradiation is stopped. Further, when it is detected that the state detecting no person by the biosensor 11 has continued for a non-irradiation time T3, even when switching from a state detecting a person by the biosensor 11 to a state detecting no person is not detected, the UVC-LED 12a is driven to perform ultraviolet irradiation for the irradiation time T2. As illustrated in FIG. 5B, when the presence of a person is detected by the biosensor 11 while periodically performing ultraviolet irradiation every time a state detecting no person has continued for the non-irradiation time T3 or while performing ultraviolet irradiation by detecting the disappearance of a person, the ultraviolet irradiation is immediately stopped. When performing ultraviolet irradiation, the UVC-LED 12a is driven and simultaneously the blue LED 12b is driven in such a way that blue light of the blue LED 12b is irradiated on the same area as an ultraviolet irradiation area by the UVC-LED 12a. This makes it possible to visualize the ultraviolet irradiation area.

The waiting time T1 is set to a time long enough to be able to regard that the state has switched to a state detecting no person, based on a detection signal of the biosensor 11, and is set to, for example, about 5 seconds. The irradiation time T2 is set according to an irradiation time required for sufficiently sterilizing an irradiation target by ultraviolet irradiation of the UVC-LED 12a, and is set to, for example, about 10 minutes. The non-irradiation time T3 is set to, for example, about 50 minutes. That is, even in a state detecting no person by the biosensor 11, for example, even in a state where no one has operated an ATM of a bank or the like after performing ultraviolet irradiation last time, sterilization is periodically performed by periodically performing ultraviolet irradiation.

In this way, ultraviolet irradiation is performed based on a detection signal of the biosensor 11, and since there is a possibility that someone has operated the ATM or the like when switching to a state detecting no person is detected based on the biosensor 11, it is possible to efficiently sterilize the irradiation target by performing ultraviolet irradiation to perform sterilization.

Further, since ultraviolet irradiation is not performed at a time point of switching to a state where the biosensor 11 detects no person, but is performed at a time point at which the waiting time T1 has elapsed, it is configured that the ultraviolet irradiation is performed on or after a time point when a state is achieved in which it is possible to regard that a person has moved to the outside of the irradiation range of ultraviolet light, and therefore it is possible to prevent the exposure more reliably and thus improve the safety.

Further, since ultraviolet irradiation is performed when a state detecting no person has continued for the non-irradiation time T3 after performing ultraviolet irradiation on an irradiation target, even when an operation of the ATM or the like is not performed continuously, sterilization is periodically performed by periodically performing ultraviolet irradiation, and therefore it is possible to keep the ATM or the like in a state where a certain sterilization effect is obtained.

It is not necessary to perform ultraviolet irradiation every time the non-irradiation time T3 has elapsed, i.e., periodically, and ultraviolet irradiation may be performed intermittently. Further, when periodically performing ultraviolet irradiation every time the non-irradiation time T3 has elapsed, since no one has operated the ATM or the like, the irradiation time of ultraviolet light may be set to a time shorter than the irradiation time T2.

Further, since it is possible to visualize an ultraviolet irradiation area by the blue LED 12b, a person who operates the ATM or the like can easily recognize the ultraviolet irradiation area so that it is possible to prevent the exposure more reliably.

Since the biosensor 11 senses the heat, when the temperature around the biosensor 11 is increased, the detection accuracy is reduced. In the ultraviolet irradiation apparatus 1 according to the first embodiment, the sensor board 11a mounted with the biosensor 11 and the light emitting boards 12e mounted with the UVC-LEDs 12a and the blue LEDs 12b are disposed to be spaced apart from each other, and further, the heat sinks 12c are fixed to the light emitting boards 12e. The heat dissipation effect of the UVC-LEDs 12a is enhanced by the heat sinks 12c. By fixing the heat sinks 12c to the rear surface cover 3c and fixing the biosensor 11 to the light shielding cover 3b so as to dispose the biosensor 11 to be spaced apart as much as possible from the heat sinks 12c to which the heat of the UVC-LEDs 12a is transferred, it is possible to further reduce the influence of the heat.

Since the ultraviolet irradiation area is visualized, a person can easily recognize the ultraviolet irradiation area visually. Therefore, it is possible to easily perform the installation work or the like of the ultraviolet irradiation apparatus 1. Further, by the visualization of the ultraviolet irradiation area, a person who operates the ATM or the like as the irradiation target, for example, can recognize whether or not sterilization is properly performed, and can recognize that ultraviolet irradiation is not performed when operating it. Therefore, it is possible to obtain a sense of security.

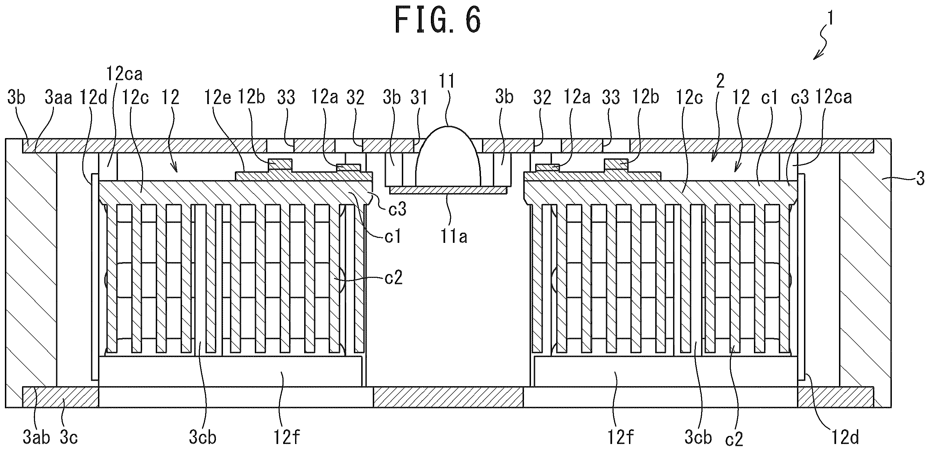

Herein, the description has been given of the case where the heat dissipation effect is obtained by providing the heat sinks 12c, but not limited thereto. Cooling fans may be provided along with the heat sinks 12c, and as illustrated in FIG. 6, cooling fans 12f may be provided between the heat sinks 12c and the rear surface cover 3c.

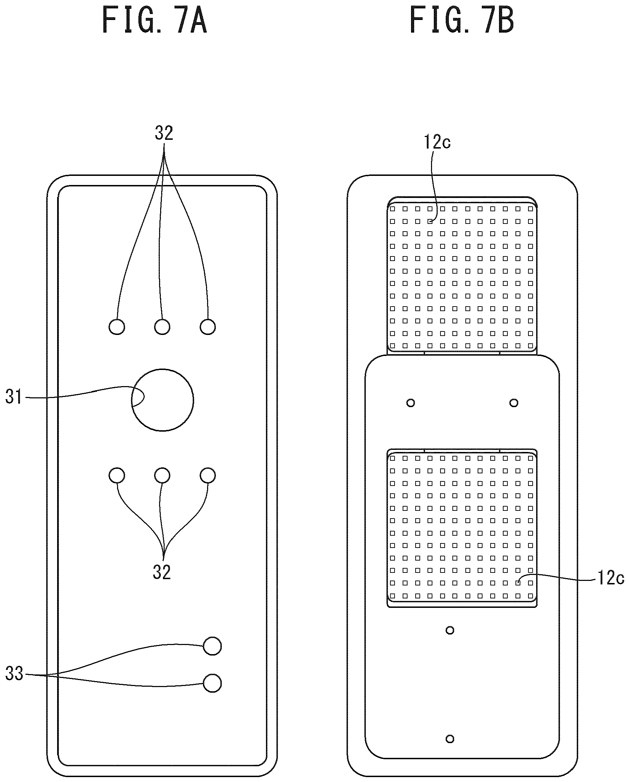

In the first embodiment described above, the description has been given of the case where the biosensor 11, the UVC-LEDs 12a, and the blue LEDs 12b are disposed as illustrated in FIG. 3A, but not limited thereto, i.e., they can be disposed at arbitrary positions . As illustrated in FIG. 7A, the blue LEDs 12b (corresponding to the holes 33) can be disposed close to one end of the outer case 3 in the longitudinal direction, and the biosensor 11 (corresponding to the hole 31) and the UVC-LEDs 12a (corresponding to the holes 32) can be disposed slightly closer to the other end of the outer case 3 from the center of the outer case 3 in the longitudinal direction. In FIGS. 7A and 7B, FIG. 7A is a front view and FIG. 7B is a rear view.

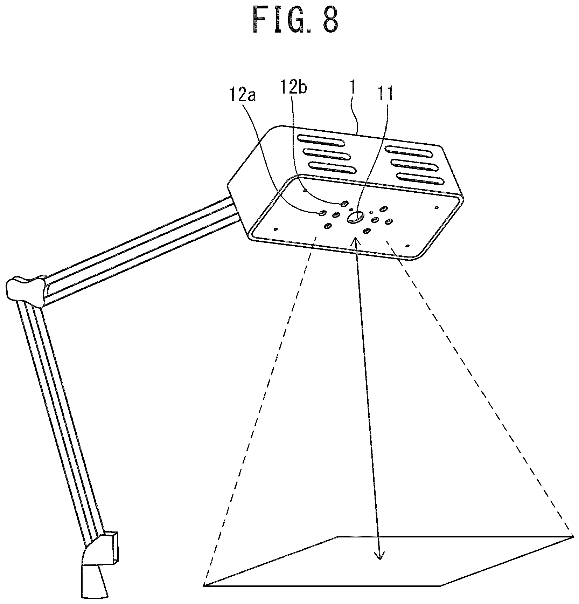

In the first embodiment described above, the ultraviolet irradiation apparatus 1 may be fixed solely at a position where it is possible to perform ultraviolet irradiation on an irradiation target. Alternatively, for example, as illustrated in FIG. 8, the ultraviolet irradiation apparatus 1 may be attached to a distal end of a movable arm so as to be installed as a desk light type ultraviolet irradiation apparatus.

Alternatively, the apparatus body 2 may be embedded in a wall or the like, and the holes 31 to 33 may be formed in a wall plate at positions facing the biosensor 11, the UVC-LEDs 12a, and the blue LEDs 12b to provide the wall plate with a configuration corresponding to the light shielding cover 3b, thereby realizing the functional configuration equivalent to the ultraviolet irradiation apparatus 1.

<Second Embodiment>

Next, a second embodiment of the present invention will be described.

An ultraviolet irradiation apparatus 1 according to the second embodiment defines the positional relationship between the UVC-LEDs 12a and the biosensor 11 in the ultraviolet irradiation apparatus 1 according to the first embodiment.

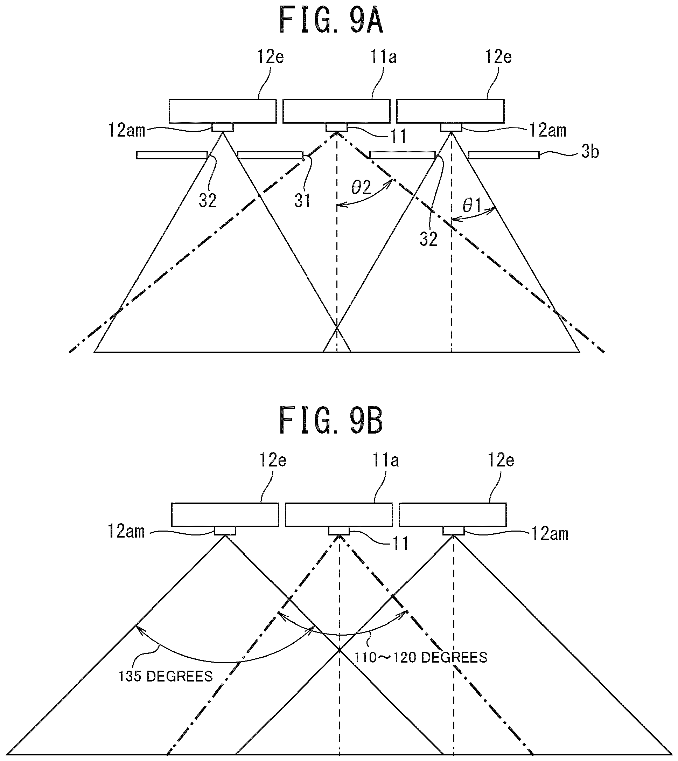

FIG. 9A illustrates a sensing area (detection range) of the biosensor 11 and irradiation areas (irradiation ranges) of the UVC-LEDs 12a. In FIG. 9A, as illustrated in FIG. 1B, a UVC-LED 12am is, out of the three UVC-LEDs 12a mounted on each of the light emitting boards 12e, the UVC-LED 12a located at a position farthest from the biosensor 11.

As illustrated in FIG. 9A, the ultraviolet irradiation apparatus 1 according to the second embodiment is configured such that the sensing area of the biosensor 11 is larger than the sum of the irradiation areas of the two UVC-LEDs 12am. That is, for example, as illustrated in FIG. 9B, in the case where the sensing area of the biosensor 11 is smaller than the sum of the irradiation areas of the two UVC-LEDs 12am, when a person approaches an irradiation target (e.g., ATM) of ultraviolet light to operate the irradiation target, the person enters the irradiation area before entering the sensing area of the biosensor 11. Therefore, when the entry of the person into the sensing area is detected by the biosensor 11 while ultraviolet irradiation is performed by the UVC-LEDs 12am, the ultraviolet irradiation is stopped after the person is exposed. Even in the case where ultraviolet irradiation is not being performed, when the timing to periodically perform ultraviolet irradiation comes, the ultraviolet irradiation is performed at this time point so that the person is exposed, and thereafter, at a time point when the entry of the person into the sensing area is detected by the biosensor 11, the ultraviolet irradiation is stopped. Since the UVC-LED is generally regarded as a point light source, an ultraviolet radiation angle of the UVC-LED 12am in FIG. 9B is exemplarily illustrated, and actually, ultraviolet light is radiated at an angle greater than the illustrated angle.

On the other hand, in the ultraviolet irradiation apparatus 1 according to the second embodiment, at a stage before a person enters the irradiation area, the presence of the person is detected so that ultraviolet irradiation is stopped and thus is not performed, and therefore it is possible to prevent the exposure more reliably and thus improve the safety.

Herein, in the ultraviolet irradiation apparatus 1 according to the second embodiment, in order to make the sensing area of the biosensor 11 larger than the irradiation area of the UVC-LED 12am, the sensing area and the irradiation area are adjusted. Specifically, out of ultraviolet light emitted from the UVC-LED 12am, a maximum angle formed by an emission line being the ultraviolet light passing through the hole 32 and emitted toward an irradiation target without being shielded by the light shielding cover 3b, and a normal vector of a light emitting surface of the UVC-LED 12am is given by a maximum radiation angle equivalent value $\Theta 1$. Further, an angle half a maximum detection angle detectable by the biosensor 11 in the state where the biosensor 11 is disposed in the outer case 3, that is, a maximum detection angle being a maximum value of a detection angle detectable by the biosensor 11 in the state where the biosensor 11 is incorporated in the ultraviolet irradiation apparatus 1, is given by a viewing angle equivalent value $\Theta 2$. Then, the sensing area and the irradiation area are set so that the maximum radiation angle equivalent value $\theta 1$ becomes smaller than the viewing angle equivalent value $\theta 2$ ($\theta 1 < \theta 2$).

By the setting described above, since the expansion rate of the sensing area of the biosensor 11 is greater than the expansion rate of the irradiation area of the UVC-LED 12am when the distance between the ultraviolet irradiation apparatus 1 and the ultraviolet irradiation target is increased, the ratio of the irradiation areas of the two UVC-LEDs 12am included in the sensing area increases as the distance between the ultraviolet irradiation apparatus 1 and the ultraviolet irradiation target increases so that the irradiation areas of the two UVC-LEDs 12am are both included in the sensing area soon.

Therefore, by performing the adjustment so that the maximum radiation angle equivalent value $\Theta 1$ of the UVC-LED 12am becomes smaller than the viewing angle equivalent value $\theta 2$ ($\theta 1 < \theta 2$), it is possible to include the irradiation areas of the two UVC-LEDs 12am in the sensing area. That is, when a person approaches the irradiation target, first, the presence of the person entering the sensing area is detected so that the driving of the UVC-LEDs 12am is stopped to stop ultraviolet irradiation, and thereafter, the person enters the ultraviolet irradiation area. That is, the possibility that the person entering the ultraviolet irradiation area is exposed is reduced.

Further, for example, in use for sterilizing a surface of a touch screen or the like, if even a person passing nearby is detected, this leads to a malfunction, and therefore it is desirable to detect the entry of a person limited to within an irradiation area. In this configuration, by adjusting the opening diameters of the hole 31 and the holes 32 in the light shielding cover 3b so as to achieve $\theta 2 < \theta 1 \times 2$, it is possible to prevent false detection by the biosensor due to expanding the detection area with respect to the irradiation areas more than necessary.

These adjustments are performed by adjusting, for example, any one of or a combination of a plurality of the opening diameters and the arrangement positions of the hole 31 and the holes 32, the distance from the light receiving surface of the biosensor 11 to the hole 31, the distance from the light emitting surface of the UVC-LED 12am to the hole 32, the viewing angle performance of the biosensor 11, the maximum angle of irradiation light of the UVC-LED 12am, and the like.

Then, further, the adjustment is performed by adjusting any one of or a combination of a plurality of the distance between the ultraviolet irradiation apparatus 1 and the irradiation target, the distance between the biosensor 11 and the UVC-LED 12am, and the like so that the irradiation areas of the two UVC-LEDs 12am are included in the sensing area on the irradiation target.

Herein, in the ultraviolet irradiation apparatus 1 according to the second embodiment, it is set that the irradiation areas of the two UVC-LEDs 12am each with the maximum distance from the biosensor 11 are included in the sensing area of the biosensor 11. Therefore, out of the UVC-LEDs 12a, the detection areas of the UVC-LEDs 12a each with a distance from the biosensor 11 shorter than the distance between the UVC-LED 12am and the biosensor 11 are invariably included in the sensing area of the biosensor 11.

As described above, the relationship between the sensing area and the irradiation areas changes depending on the distances between the biosensor 11 and the UVC-LEDs 12a, and therefore, differently from FIG. 1B in which the three UVC-LEDs 12a are disposed at regular intervals in two rows sandwiching the biosensor 11, the UVC-LEDs 12a may be disposed on a circle centered at the biosensor 11 so that the distances from the biosensor 11 are equal to each other.

<Third Embodiment>

Next, a third embodiment of the present invention will be described.

An ultraviolet irradiation apparatus 1 according to the third embodiment is provided with a side cover in the ultraviolet irradiation apparatus 1 according to the second embodiment.

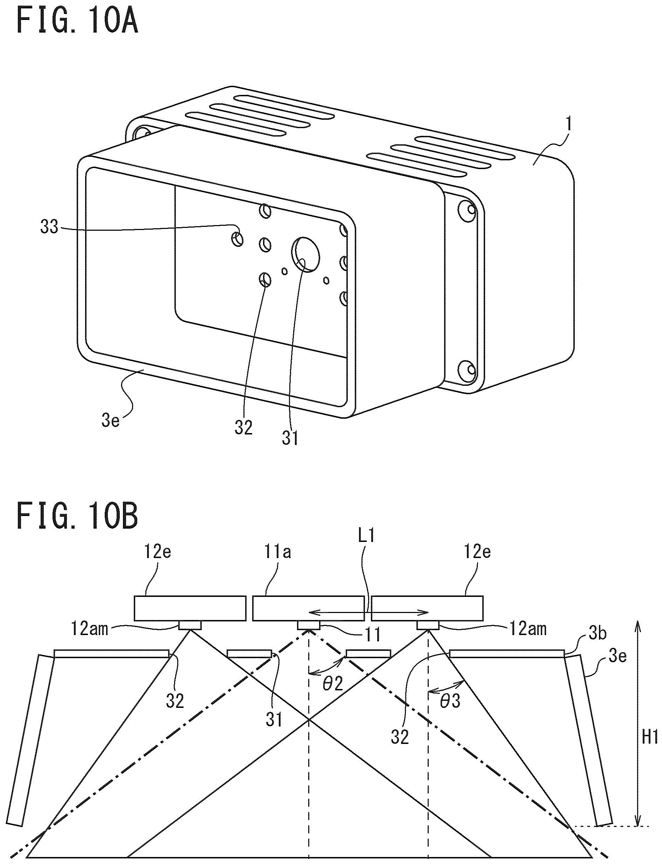

FIGS. 10A and 10B illustrate the ultraviolet irradiation apparatus 1 according to the third embodiment, wherein FIG. 10A is a perspective view illustrating an example of the ultraviolet irradiation apparatus including the side cover, and FIG. 10B is a sectional view illustrating the main part.

In the ultraviolet irradiation apparatus 1 according to the third embodiment, as illustrated in FIG. 10A, a side cover (protruding portion) 3e surrounding the holes 31 to 33 corresponding to the biosensor 11, the UVC-LEDs 12a, and the blue LEDs 12b is provided on the surface of the light shielding cover 3b on the side opposite to the rear surface cover 3c. The side cover 3e has an end surface formed in a hollow rectangular parallelepiped shape, for example. The side cover 3e is not limited to the hollow rectangular parallelepiped shape, and in the sectional view illustrated in FIG. 10B, the side cover 3e may have a bulb umbrella shape such that its end surface is inclined outward and becomes larger as away from the light shielding cover 3b.

The side cover 3e is set so that a height H1 along a normal vector of a light emitting surface of the UVC-LED 12a from the light emitting surface to an end of the side cover 3e on the side opposite to the light shielding cover 3b satisfies a formula (1) below.

$$H1 \geq L1 \times \cos(\theta 2) \times \cos(\theta 3) / \sin(\theta 2 - \theta 3) \quad (1)$$

In the formula (1), L1 is a distance between the UVC-LED 12am being, out of the UVC-LEDs 12a, the farthest light source disposed at the position farthest from the biosensor 11, and the biosensor 11. $\Theta 2$ is a viewing angle equivalent value being an angle half a maximum detection angle detectable by the biosensor 11 in the state where the biosensor 11 is disposed in the outer case 3. $\theta 3$ is a farthest radiation angle equivalent value representing an angle formed by, out of emission lines being ultraviolet light emitted from the UVC-LED 12am being the farthest light source, passing through the hole 32, and emitted toward an irradiation target without being shielded by the light shielding cover 3b, the emission line passing through a point farthest from the biosensor 11 in plan view and a normal vector of a light emitting surface of the UVC-LED 12am being the farthest light source.

As illustrated in FIG. 10B, even when an irradiation area is included in a sensing area on an irradiation target, an irradiation area is not included in a sensing area in a spatial area near the light shielding cover 3b between the ultraviolet irradiation apparatus 1 and the irradiation target. Consequently, when a human hand or the like enters such a spatial area, the human hand or the like first enters the irradiation area and thereafter enters the sensing area. That is, there is a possibility of being exposed. Therefore, the side cover 3e is provided to prevent the human hand or the like from entering the spatial area where the irradiation area is not included in the sensing area. For preventing the entry into the spatial area where the irradiation area is not included in the sensing area, it is satisfactory if the human hand or the like does not enter an area from the light shielding cover 3b to a point where the sensing area of the biosensor 11 and the irradiation area of the UVC-LED 12am coincide with each other. From the formula (1), the point where the sensing area of the biosensor 11 and the irradiation area of the UVC-LED 12am coincide with each other can be given by a point of the height H1 along the normal vector from the light emitting surface of the UVC-LED 12am. Therefore, by setting that the height H1 along the normal vector of the light emitting surface of the UVC-LED 12am from the light emitting surface to the distal end of the side cover 3e satisfies the formula (1), at least, the side cover 3e higher than the point where the sensing area of the biosensor 11 and the irradiation area of the UVC-LED 12as coincide with each other is formed. As a result, even when the irradiation area is not included in the sensing area in the spatial area, the entry of the human hand or the like into the area where there is a possibility of being exposed is prevented by the side cover 3e so that it is possible to further improve the safety.

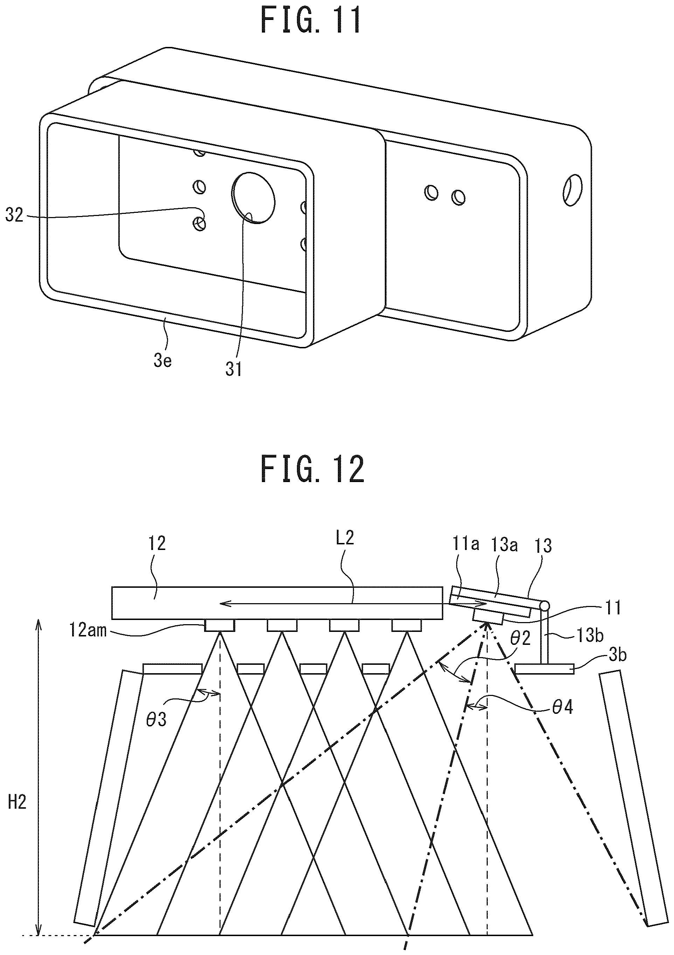

In the case where the blue LEDs 12b (corresponding to the holes 33) and the UVC-LEDs 12a (corresponding to the holes 32) are disposed to be spaced apart from each other as illustrated in FIG. 7A, the side cover 3e may be provided to surround the holes 31, 32 corresponding to the biosensor 11 and the UVC-LEDs 12a excluding the holes 33 corresponding to the blue LEDs 12b as illustrated in FIG. 11.

In this ultraviolet irradiation apparatus 1, it is not always necessary to surround the four sides by the side cover as illustrated in FIG. 10A or FIG. 11, and a cover may be provided only in the direction in which there is a possibility of access of a person. For example, when it is supposed that the ultraviolet irradiation apparatus 1 is disposed above a touch screen of an ATM, it is sufficient to assume only access of a person from the direction of this side of the apparatus, and therefore covers on the far side and the lateral sides of the apparatus are unnecessary.

<Fourth Embodiment>

Next, a fourth embodiment of the present invention will be described.

The fourth embodiment is configured such that the side cover 3e is provided and the arrangement positions of the biosensor 11 and the UVC-LEDs 12a are changed in the ultraviolet irradiation apparatus 1 according to the first embodiment.

The ultraviolet irradiation apparatus 1 according to the fourth embodiment includes the single light emitting unit 12 and the single biosensor 11 as illustrated in FIG. 12. The biosensor 11 is disposed on one end portion side in the longitudinal direction as viewed from the light shielding cover 3b side, and the UVC-LEDs 12a are disposed on the other end portion side in the longitudinal direction from the biosensor 11. Further, the biosensor 11 is provided to an inclination adjustment mechanism 13, and by adjusting its inclination by the inclination adjustment mechanism 13, the inclination of its sensing surface is directed to the UVC-LED 12a side. The inclination adjustment mechanism 13 is configured such that, for example, a support member 13a to which the sensor board 11a can be attached, and a fixing member 13b are movably fixed, that one end of the fixing member 13b is fixed on a surface of the light shielding cover 3b on the side opposite to the rear surface cover 3c, that the sensor board 11a is fixed to the support member 13a, and that, by adjusting the inclination of the support member 13a manually, the inclination of the sensor board 11a is adjusted so as to adjust the inclination of the sensing surface. Herein, it is configured that, by providing the inclination adjustment mechanism 13, the inclination of the biosensor 11 can be adjusted even after the biosensor 11 is incorporated into the ultraviolet irradiation apparatus 1. However, for example, when there is no need to adjust the inclination of the biosensor after it is incorporated into the ultraviolet irradiation apparatus 1, the inclination adjustment mechanism 13 is not necessarily provided.

By performing the adjustment by the inclination adjustment mechanism 13, the sensor board 11a is arranged to be inclined so that the sensing surface is directed to the UVC-LED 12a side, and consequently, the irradiation areas of all the UVC-LEDs 12a are included in the sensing area of the biosensor 11. Although the blue LEDs 12b are not illustrated in FIG. 12, the blue LEDs 12b are arranged so that the blue irradiation areas thereof overlap the irradiation areas of all the UVC-LEDs 12a.

By arranging the biosensor 11 and the UVC-LEDs 12a in this way, the degree of freedom of arrangement position of the biosensor 11 can be improved. That is, as illustrated in FIG. 1B, in the case where the biosensor 11 is arranged at the center as viewed from the front, in order to make the arrangement so that the irradiation areas of the UVC-LEDs 12a are included in the sensing area of the biosensor 11, it is necessary to arrange the UVC-LEDs 12a around the biosensor 11. On the other hand, as illustrated in FIG. 12, in the case where all the UVC-LEDs 12a are arranged on one side of the biosensor 11, it is sufficient to arrange the biosensor 11 to be inclined to the UVC-LED 12a side so that the irradiation areas of these UVC-LEDs 12a are included in the sensing area. That is, since it is sufficient to arrange the biosensor 11 at any position around the plurality of UVC-LEDs 12a, it is possible to improve the degree of freedom of arrangement of the biosensor 11.

Like in the ultraviolet irradiation apparatus 1 according to the third embodiment, the side cover 3e is provided on the light shielding cover 3b. The height of the side cover 3e can be given a formula (2) below as a distance H2 along a normal vector of a light emitting surface of the UVC-LED 12a from the light emitting surface of the UVC-LED 12a to an end of the side cover 3e on the side opposite to the light shielding cover 3b.

$$H2 \geq L2 \times \cos(\theta2 + \theta4) \times \cos(\theta3) / \sin(\theta4 + \theta2 - \theta3) \quad (2)$$

In the formula (2), L2 is a distance between the UVC-LED 12am being, out of the UVC-LEDs 12a, the farthest light source disposed at the position farthest from the biosensor 11, and the biosensor 11. $\Theta 2$ is a viewing angle equivalent value being an angle half a maximum detection angle detectable by the biosensor 11 in the state where the biosensor 11 is disposed in the outer case 3. $\theta 3$ is a farthest radiation angle equivalent value representing an angle formed by, out of emission lines being ultraviolet light emitted from the UVC-LED 12am being the farthest light source, passing through the hole 32, and emitted toward an irradiation target without being shielded by the light shielding cover 3b, the emission line passing through a point farthest from the biosensor 11 in plan view and a normal vector of a light emitting surface of the UVC-LED 12am being the farthest light source. $\theta 4$ is an inclination of the sensing surface of the biosensor 11.

By inclining the sensing surface of the biosensor 11, a point where the irradiation area of the UVC-LED 12am located at the position farthest from the biosensor 11 and the sensing area of the biosensor 11 coincide with each other becomes a position closer to the light shielding cover 3b compared to the case where the biosensor 11 is not inclined. Therefore, H2 corresponding to the height of the side cover 3e along the normal vector of the light emitting surface of the UVC-LED 12am being the farthest light source can be shortened by a value corresponding the inclination of the biosensor 11, i.e., the height of the side cover 3e can be lowered. That is, the ultraviolet irradiation apparatus 1 can be further reduced in size. In this case, the arrangements of the holes 31 to 33 of the light shielding cover 3b corresponding to the biosensor 11, the UVC-LEDs 12a, and the blue LED 12b may be changed so as to match the arrangements of the biosensor 11, the UVC-LEDs 12a, and the blue LED 12b.

<Fifth Embodiment>

Next, a fifth embodiment of the present invention will be described.

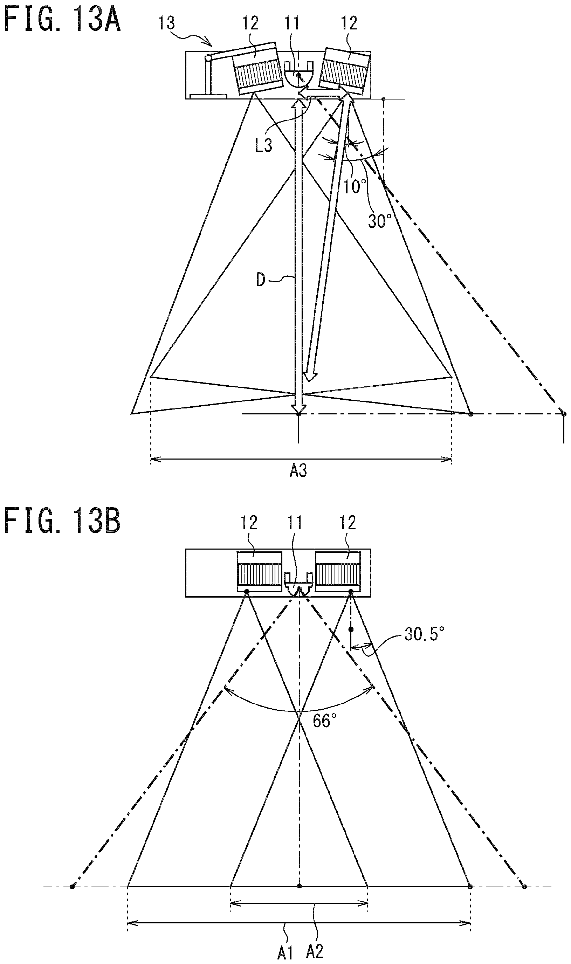

The fifth embodiment is configured such that the arrangement positions of the light emitting units 12 are changed in the ultraviolet irradiation apparatus 1 according to the first embodiment. In the ultraviolet irradiation apparatus 1 according to the fifth embodiment, as illustrated in FIG. 13A, the two light emitting units 12 are respectively disposed so that the light emitting surfaces of the UVC-LEDs 12a are inclined to the biosensor 11 side. The light emitting unit 12 is set so that an inclination (inclination angle) $\theta a$ of the light emitting surface of the UVC-LED 12a satisfies a formula (3) below.

$$\theta a = a \sin (L3 / D) \quad (3)$$

Assuming that the UVC-LED 12a disposed at a position closest to the light shielding cover 3b out of the plurality of UVC-LEDs 12a mounted in each of the light emitting units 12 is given by a UVC-LED 12an, L3 in the formula (3) represents a distance between the biosensor 11 and the UVC-LED 12an, and D represents a distance between the light emitting surface of the UVC-LED 12an and an irradiation target.

By arranging in this way, as illustrated in FIG. 13A, the irradiation areas of the UVC-LEDs 12a respectively mounted in the two light emitting units 12 overlap each other to increase the irradiation density. That is, in the case where, like in the ultraviolet irradiation apparatus 1 according to the first embodiment, the light emitting surfaces of the UVC-LEDs 12a mounted in the two light emitting units 12 disposed to sandwich the biosensor 11 face in the same direction, as illustrated in FIG. 13B, although an irradiation area A1 is expanded, an area A2 where the irradiation areas by the UVC-LEDs 12a mounted in the two light emitting units 12 overlap each other is narrow. Therefore, an area where the irradiation density is high is narrower compared to an area where the irradiation density is high in the case where an area A3 where the irradiation areas overlap each other, illustrated in FIG. 13A is large.

Therefore, for example, by disposing the two light emitting units 12 so as to be inclined in the directions facing each other, it is possible to easily improve the sterilization performance.

The inclination adjustment mechanism (light source inclination adjustment mechanism) 13 illustrated in FIG. 12 may be applied to the light emitting unit 12, and the inclination of the light emitting unit 12 may be adjusted manually. The inclination adjustment mechanism 13 may be provided to each of the two light emitting units 12 or may be provided to only one of them. By mounting one or a plurality of UVC-LEDs 12a in each of the light emitting units 12, the inclinations of the UVC-LEDs 12a can be adjusted one by one or per the plurality.

<Sixth Embodiment>

Next, a sixth embodiment of the present invention will be described.

The sixth embodiment is configured such that a plurality of, for example, two, biosensors 11-a, 11-b are provided in the ultraviolet irradiation apparatus 1 according to the first embodiment.

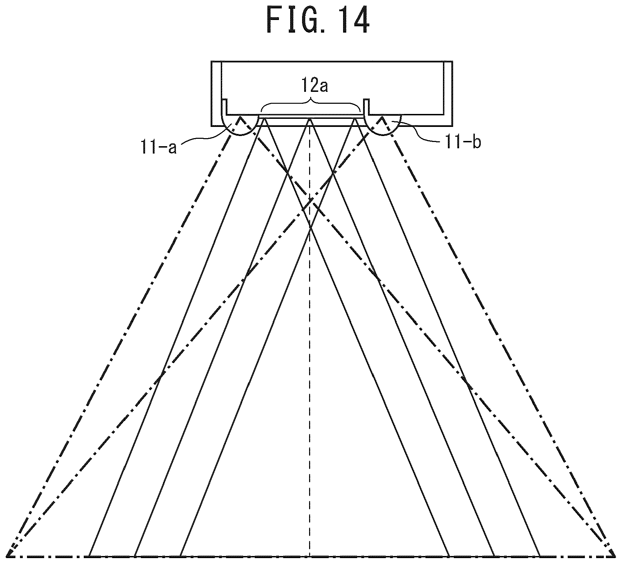

That is, as illustrated in FIG. 14, the UVC-LEDs 12a are arranged collectively and the two biosensors 11-a, 11-b are provided around these UVC-LEDs 12a. In this event, the biosensors 11-a, 11-b may be disposed so that sensing surfaces thereof are inclined to the UVC-LED 12a side, or may be disposed so that the sensing surfaces are parallel to the light emitting surfaces of the UVC-LEDs 12a. Further, the number of the biosensors 11 provided may be three or more.

By providing the plurality of biosensors 11 in this way, an area sensing a person is expanded. Therefore, it is possible to sense the presence of a person more reliably.

In the case where the plurality of biosensors 11 is provided, the same type of sensor may be provided, or, for example, a plurality of different type sensors may be provided. Depending on a use and an installation environment of the ultraviolet irradiation apparatus 1, the biosensors 11 may be selected.

<Seventh Embodiment>

Next, a seventh embodiment of the present invention will be described.

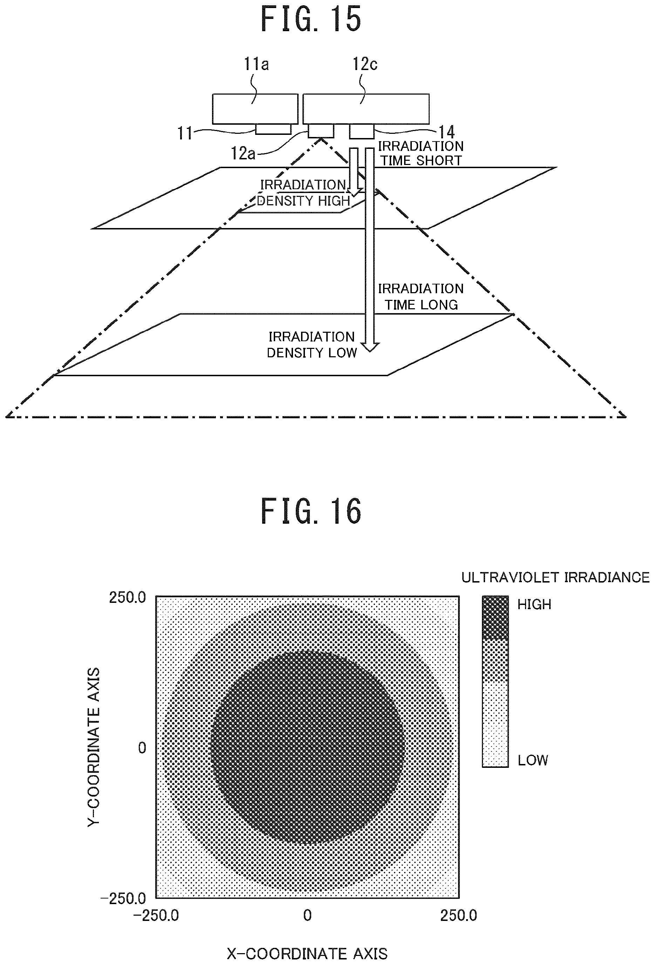

The seventh embodiment is configured such that, as illustrated in FIG. 15, a distance sensor 14 is further provided in the ultraviolet irradiation apparatus 1 according to the first embodiment.

The distance sensor 14 is mounted on the light emitting board 12e mounted with the UVC-LEDs 12a and measures the distance to an object that is present in the same direction as the light emitting surfaces of the UVC-LEDs 12a.

Then, the control circuit 1a controls the driving time of the UVC-LEDs 12a based on a detection signal of the distance sensor 14 in such a way that the longer the distance to an irradiation target, the longer the irradiation time of ultraviolet light.

That is, as illustrated in FIG. 15, the longer the distance to the irradiation target, the lower the irradiation density. Therefore, by increasing the irradiation time as the distance to the irradiation target gets longer, it is possible to give a fixed irradiation intensity (irradiation density $\times$ time) to the irradiation target. Therefore, it is possible to obtain stable sterilization performance for the irradiation target.

Further, by recognizing that a person has entered an irradiation area when a differential value of a measured distance exceeds a threshold value, the distance sensor can also be used as a biosensor. By using the distance sensor as the biosensor along with the biosensor 11, the redundancy is increased so that it is possible to detect the entry of a person more reliably.

Not limited to the case where the distance sensor 14 is provided in the ultraviolet irradiation apparatus 1 according to the first embodiment, it is also possible to provide the distance sensor 14 in the second to fifth embodiments, and similar actions and effects can be obtained.

In each of the embodiments described above, the description has been given of the case where the two blue LEDs 12b are provided, but not limited to the two, and one or three or more may be provided. Likewise, the number of the UVC-LEDs 12a is not limited to six, and a desired number of the UVC-LEDs 12a may be provided. For example, when high sterilization performance is required, or when wishing sterilization in a shorter time, a larger number of the UVC-LEDs 12a may be provided.

In each of the embodiments described above, the detection angle of the biosensor 11 may be configured not to be narrowed by the hole 31 formed in the light shielding cover 3b. Further, the biosensor 11 may be provided outside the outer case 3, i.e., on the front surface of the light shielding cover 3b.

Likewise, when embedding the apparatus body 2 in a wall or the like, the biosensor 11 may be fixed to the surface of the wall.

When the detection angle of the biosensor 11 is not narrowed by the hole 31 formed in the light shielding cover 3b, or when the detection angle of the biosensor 11 is not narrowed by attaching the biosensor 11 to the front surface of the light shielding cover 3b or attaching the biosensor 11 to the surface of the wall, a viewing angle determined by the specification of the biosensor 11 is a maximum detection angle, and an angle half the viewing angle is a viewing angle equivalent value Θ2.

While the embodiments of the present invention have been described above, the embodiments described above merely exemplify apparatus and methods for embodying the technical idea of the present invention, and the technical idea of the present invention does not specify materials, shapes, structures, arrangements, and the like of constituent components. The technical idea of the present invention can be changed in various ways within the technical scope defined by the claims.

Example

Using the ultraviolet irradiation apparatus 1 according to the first embodiment, a simulation was conducted in which ultraviolet irradiation was performed on a 500 mm × 500 mm irradiation target located at a position spaced apart from the light shielding cover 3b by 300 mm. A case was assumed where the output of the UVC-LED 12a was 70 mW and the six UVC-LEDs 12a were driven by 465 mA. As a result, as illustrated in FIG. 16, a high ultraviolet irradiance was obtained at an approximately central portion of an irradiation area. In FIG. 16, at a portion where the ultraviolet irradiance was the highest, the dose amount (cumulative amount of light (UV exposure amount)) was 40 mj/cm$^2$ or more for 10 minutes.

Reference Signs List

| | |
|---|---|
| 1 | ultraviolet irradiation apparatus |
| 1a | control circuit |
| 2 | apparatus body |
| 3 | outer case |
| 3b | light shielding cover |
| 3c | rear surface cover |
| 3e | side cover |
| 11 | biosensor |
| 11a | sensor board |
| 12 | light emitting unit |
| 12a | UVC-LED |
| 12b | blue LED |
| 12c | heat sink |
| 12d | driver board |
| 12e | light emitting board |

**Claims**

**1.** An ultraviolet irradiation apparatus comprising:

a light source configured to emit ultraviolet light;
a biosensor disposed in a vicinity of the light source such that an irradiation range of the light source and a detection range of the biosensor overlap each other, the biosensor configured to detect a presence of a person;
an electronic circuit configured to control the light source based on an output signal of the biosensor; and
a light shielding cover configured to shield a part of the ultraviolet light emitted from the light source,
wherein:

the light source is disposed in an area on a side opposite to one side where an irradiation target of the ultraviolet light is present, the light source and the irradiation target sandwiching the light shielding cover;
a sensing surface of the biosensor and a light emitting surface of the light source are disposed to face in a same direction;
an angle Θ2 half a maximum detection angle of the biosensor in a state where the biosensor is incorporated in the ultraviolet irradiation apparatus is greater than a maximum angle Θ1 formed by a normal vector of the light emitting surface and the ultraviolet light emitted from the light source and passing without being shielded by the light shielding cover; and
the electronic circuit is configured to control the light source to stop emission of the ultraviolet light when the biosensor has detected the presence of the person, the electronic circuit configured to control the light source to emit the ultraviolet light again when a state has switched from a state where the biosensor is detecting the presence of the person to a state where the biosensor does not detect the presence of the person.

**2.** The ultraviolet irradiation apparatus according to claim 1, wherein the angle $\theta 2$ is smaller than an angle twice the maximum angle $\Theta 1$.

**3.** The ultraviolet irradiation apparatus according to claim 1 or 2, including:

one or a plurality of the light sources; and
a protruding portion provided to protrude to the one side from the light shielding cover to surround ultraviolet light emitted from the one or the plurality of light sources and passing through the light shielding cover,
wherein, assuming that a distance between, out of the light sources, the farthest light source disposed at a position farthest from the biosensor, and the biosensor is given by L1, and
that an angle formed by a normal vector of a light emitting surface of the farthest light source and, out of the ultraviolet light emitted from the farthest light source and passing through the light shielding cover, the ultraviolet light passing through a point farthest from the biosensor in plan view is given by $\theta 3$,
a distance along the normal vector of the light emitting surface of the light source between the light emitting surface and a distal end of the protruding portion is equal to or more than

$$L1 \times \cos(\theta 2) \times \cos(\theta 3) / \sin(\theta 2 - \theta 3).$$

**4.** The ultraviolet irradiation apparatus according to claim 1, including:

one or a plurality of the light sources;
the biosensor provided to be inclined to the light source side; and
a protruding portion provided to protrude to the one side from the light shielding cover to surround ultraviolet light emitted from the one or the plurality of light sources and passing through the light shielding cover,
wherein, assuming that a distance between, out of the light sources, the farthest light source disposed at a position farthest from the biosensor, and the biosensor is given by L2,
that an angle formed by a normal vector of a light emitting surface of the farthest light source and, out of the ultraviolet light emitted from the farthest light source and passing through the light shielding cover, one ultraviolet light passing through a point farthest from the biosensor in plan view is given by $\theta 3$, and
that an inclination angle of a normal vector of the sensing surface of the biosensor toward the side of the one ultraviolet light is given by $\theta 4$,
a distance along the normal vector of the light emitting surface of the light source between the light emitting surface and a distal end of the protruding portion is equal to or more than

$$L2 \times \cos(\theta 2 + \theta 4) \times \cos(\theta 3) / \sin(\theta 4 + \theta 2 - \theta 3).$$

**5.** The ultraviolet irradiation apparatus according to claim 1 or 2, wherein a plurality of the light sources is disposed around the biosensor.

**6.** The ultraviolet irradiation apparatus according to claim 5, wherein at least one of the plurality of light sources has a light source inclination adjustment mechanism configured to incline a normal vector of a light emitting surface of the at least one light source.

**7.** The ultraviolet irradiation apparatus according to claim 6,

wherein, assuming that a distance between, out of the light sources, the farthest light source disposed at a position farthest from the biosensor, and the biosensor is given by L3, and
that a distance of a straight line from a point of intersection with a surface of the light shielding cover on the one side to a point of intersection with the irradiation target is given by D, the straight line obtained by extending a normal vector passing through a center of the sensing surface of the biosensor,
an inclination angle of the normal vector of the light emitting surface to the biosensor side is a sin (L3/D).

**8.** The ultraviolet irradiation apparatus according to any one of claims 1 to 4, wherein a plurality of the biosensors is disposed around the light source.

**9.** The ultraviolet irradiation apparatus according to any one of claims 1 to 8, comprising a heat sink thermally connected to the light source.

**10.** The ultraviolet irradiation apparatus according to claim 9, wherein the heat sink is not thermally connected to the biosensor.

**11.** The ultraviolet irradiation apparatus according to claim 9 or 10, further comprising a cooling fan.

**12.** The ultraviolet irradiation apparatus according to any one of claims 1 to 11, comprising a distance sensor configured to detect a distance to an object present in a same direction as a direction of the light emitting surface of the light source, wherein the electronic circuit is configured to control an emission time of the ultraviolet light by the light source according to a detection distance detected by the distance sensor.

**13.** The ultraviolet irradiation apparatus according to any one of claims 1 to 12, comprising a visible light source including the irradiation range of the light source in a visible light irradiation range of the visible light source.

**14.** An ultraviolet irradiation method including:

a light source configured to emit ultraviolet light; and
a biosensor disposed in a vicinity of the light source such that an irradiation range of the light source and a detection range of the biosensor overlap each other, the biosensor configured to detect a presence of a person, the ultraviolet irradiation method comprising:

disposing the light source in an area on a side opposite to one side where an irradiation target of the ultraviolet light is present, the light source and the irradiation target sandwiching a light shielding cover configured to shield a part of the ultraviolet light emitted from the light source, and disposing a sensing surface of the biosensor and a light emitting surface of the light source to face in a same direction; and
shielding the ultraviolet light such that an angle half a maximum detection angle of the biosensor in a disposed state is greater than a maximum angle formed by a normal vector of the light emitting surface of the light source and the ultraviolet light emitted from the light source and passing without being shielded by the light shielding cover.

FIG. 1A

A
33
32
31
1
32
33
3b
3
3d
A'
3da
3a

FIG. 1B

12d
12ca
12
2
12b
12ca
12a (12am)
12e
1
11a
12a
12b
12e
12
3b
12ca
3
3a
12c
3cb
12a
12a
3ba
12a (12am)
12d
3cb
12c
12ca

FIG. 2
1
A'
A
3
12d
12ca
c3
c1
12
2
c2
12c
3cb
12b
33
12a
32
3b
11
11a
31
c3
c1
c2
32
12a
33
12b
3cb
12e
12
12ca
12c
12d
3aa
3c
3ab
3b

FIG. 3A
33
32
32
33
3a
3b
32
31
32
FIG. 3B
3cb
3ca
3ca
3cb
3c
12c
3a
3cb
12c
3cb
FIG. 3C
3da
3
3a
3d

1
1a
CONTROL CIRCUIT
12aa
12ba
12aa
12ba
12
12
DRIVE CIRCUIT
DRIVE CIRCUIT
DRIVE CIRCUIT
DRIVE CIRCUIT
HUMAN SENSOR
UVC-LED
BLUE LED
UVC-LED
BLUE LED
11
12a
12b
12a
12b

FIG. 4

HUMAN SENSOR
UVC-LED
T1
T2
T3
T2
T1
HUMAN SENSOR
UVC-LED
T3
T2

FIG. 5A

FIG. 5B

FIG. 6
1
3
12ca
c3
c1
12
2
12c
12b
33
12a
32
3b
11
31
11a
12d
c2
3cb
12f
12e
3aa
3ab
3c

FIG. 7A
FIG. 7B
32
12c
31
32
12c
33

FIG. 8

12a
12b
1
11

FIG. 9A

12e
11a
12e
12am
11
12am
3b
32
31
θ2
θ1
32

FIG. 9B

12e
11a
12e
12am
11
12am
135 DEGREES
110～120 DEGREES

FIG. 10A

1
33
3e
32
31

FIG. 10B

L1
12e
11a
12e
12am
11
12am
3b
3e
32
31
θ2
θ3
32
H1

FIG. 11

32
3e
31

FIG. 12

12
L2
11a
13a
13
11
12am
θ2
13b
3b
θ3
θ4
H2

FIG. 13A

13
12
11
12
L3
10°
30°
D
A3

FIG. 13B

12
11
12
30.5°
66°
A1
A2

FIG. 14

12a
11-a
11-b

FIG. 15

11a
12c
11
14
12a
IRRADIATION
TIME SHORT
IRRADIATION
DENSITY HIGH
IRRADIATION
TIME LONG
IRRADIATION
DENSITY LOW

FIG. 16

ULTRAVIOLET IRRADIANCE
HIGH
LOW
250.0
0
−250.0
−250.0
0
250.0
Y−COORDINATE AXIS
X−COORDINATE AXIS

## INTERNATIONAL SEARCH REPORT

International application No. **PCT/JP2021/025506**

**A. CLASSIFICATION OF SUBJECT MATTER**

***A61L 2/10***(2006.01)i; ***A61L 2/24***(2006.01)i; ***H01L 33/00***(2010.01)i
FI: A61L2/10; H01L33/00 L; A61L2/24

According to International Patent Classification (IPC) or to both national classification and IPC

**B. FIELDS SEARCHED**

Minimum documentation searched (classification system followed by classification symbols)

A61L2/10; A61L2/24; H01L33/00

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2017-094090 A (VIOLET DEFENSE TECHNOLOGY, INC.) 01 June 2017 (2017-06-01) claims 1, 3-4, paragraph [0024] | 1-14 |
| A | US 2017/0296686 A1 (UV PARTNERS INC.) 19 October 2017 (2017-10-19) fig. 1, paragraphs [0005], [0053]-[0054], [0102]-[0114] | 1-14 |
| A | CN 110585457 A (GREE ELECTRIC APPLIANCES, INC. OF ZHUHAI) 20 December 2019 (2019-12-20) paragraphs [0002]-[0003], [0006]-[0007], [0067] | 1-14 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **28 September 2021** | **05 October 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2021/025506**

| Patent document cited in search report | Publication date (day/month/year) | Patent family member(s) | Publication date (day/month/year) |
|---|---|---|---|
| JP 2017-094090 A | 01 June 2017 | US 2016/0151521 A1<br>paragraph [0036], claims 1, 5, 7<br>WO 2014/078324 A1<br>EP 2919820 A1<br>KR 10-2015-0080624 A | |
| US 2017/0296686 A1 | 19 October 2017 | WO 2015/184428 A1<br>EP 3148595 A1 | |
| CN 110585457 A | 20 December 2019 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2017029293 A